Europäisches Patentamt

European Patent Office

Office européen des brevets

(19)

(11) Numéro de publication: **0 045 267**
**B1**

(12) **FASCICULE DE BREVET EUROPEEN**

(45) Date de publication du fascicule du brevet: **14.12.83**

(51) Int. Cl.³: **A 61 B 19/02,** A 61 L 2/26

(21) Numéro de dépôt: **81401219.1**

(22) Date de dépôt: **29.07.81**

(54) Equipement de stockage de matériel chirurgical, et procédé pour sa mise en oeuvre.

(30) Priorité: **30.07.80 FR 8016793**

(43) Date de publication de la demande:
**03.02.82 Bulletin 82/5**

(45) Mention de la délivrance du brevet:
**14.12.83 Bulletin 83/50**

(84) Etats contractants désignés:
**BE DE GB IT LU NL**

(56) Documents cités:
**DE - A - 1 919 896**
**US - A - 3 762 398**
**US - A - 3 853 329**

(73) Titulaire: **Dolley, Jean Bernard**
**68 Avenue Victor Hugo**
**F-78280 Guyancourt (FR)**

(72) Inventeur: **Dolley, Jean Bernard**
**68 Avenue Victor Hugo**
**F-78280 Guyancourt (FR)**

(74) Mandataire: **Moncheny, Michel et al,**
**c/o Cabinet Lavoix 2 Place d'Estienne d'Orves**
**F-75441 Paris Cedex 09 (FR)**

Courier Press, Leamington Spa, England.

## Equipement de stockage de matériel chirurgical et procédé pour sa mise en oeuvre

La présente invention concerne un équipement de stockage de matériel chirurgical spécialement destiné à être utilisé dans un bloc opératoire chirurgical comprenant une salle d'opération dont les murs sont dépourvus de volumes intérieurs de rangement et un procédé pour la mise en oeuvre de cet équipement. Un tel équipement, qui comprend plusieurs chariots consistant en une étagère rigide adaptée pour recevoir des paniers et un plateau amovibles eten un châssis démontable est connu de DE—A—1 919 896.

Suivant un agencement à peu près classique des blocs opératoires il est prévu une zone dite "propre" qui comprend les salles d'opérations elles-mêmes, une anti-chambre de préparation pour les chirurgiens et leurs assistants, éventuellement une chambre d'anesthésie et de réanimation, et une pièce dans laquelle est stocké et manipulé le matériel stérile, dite "stérilisation côté propre" ou "stock stérile", cette pièce communiquant avec une zone dite "stérilisation côté sale" ou "côté lavage" par l'intermédiaire d'un ensemble de désinfection et de stérilisation dont l'entrée se trouve dans la zone sale.

Après avoir été stérilisé le matériel est stocké dans la ou les salles d'opérations dont au moins un mur est aménagé à cet effet et comporte des vitrines, armoires, tiroirs, catgutiers, et, éventuellement des guichets traversants.

La nécessité de prévoir des éléments de rangement a conduit à profiter des aménagements en épaisseur sur le ou les murs de la salle d'opérations pour y encastrer également certains appareils tels que le bistouri électrique, le système d'aspiration chirurgien, le négatoscope, etc.

Ces éléments de rangement et ces appareils encastrés présentent en conséquence de nombreux recoins et espaces difficilement accessibles dont l'étanchéité au formol n'est pas toujours satisfaisante et dont la désinfection pose toujours des problèmes.

En outre ces aménagements muraux sont compliqués, très longs à installer et grèvent par conséquent lourdement les en frais de construction et d'installation des blocs opératoires.

L'invention a pour but de remédier à ces inconvénients en fournissant un équipement spécialement destiné à être utilisé dans un bloc opératoire chirurgical comprenant une salle d'opération dont les murs sont dépourvus de volumes intérieurs de rangement, et un procédé pour la mise en oeuvre de cet équipement, permettant de supprimer les éléments et volumes de rangement en salles d'opérations tout en mettant à portée immédiate du chirurgien les matériels et l'instrumentation dont il a besoin pour les interventions, et de simplifier les manipulations de ces instruments et accessoires après usage et notamment lors de leur désinfection et stérilisation.

L'équipement suivant l'invention, comprend plusieurs chariots démontables adaptés pour recevoir des paniers et un plateau P, amovibles, un dispositif de montage desdits chariots, un dispositif de démontage desdits chariots et un porte-chariots déplaçable adapté pour recevoir des éléments de plusieurs desdits chariots démontés, dans des positions adjacentes de façon à présenter un encombrement minimal, et au moins un support fixe adapté pour recevoir des paniers.

L'invention a également pour objet un procédé pour la mise en oeuvre de l'équipement défini ci-dessus, dans un bloc opératoire chirurgical comportant une salle d'opération dont les murs sont dépourvus de volumes intérieurs de rangement, caractérisé en ce qu'on utilise au moins un chariot démonté et au moins un lot d'instruments et d'accessoires préconditionnés disposé dans un stock stérile situé dans la zone propre du bloc opératoire, et dans lequel, préalablement à l'intervention, on procède au montage d'au moins un chariot sur lequel on charge ledit lot préconditionné, on ramène l'ensemble après l'intervention dans une zone sale, comme connu en soi, on procède au démontage dudit chariot dans cette zone sale, on reconditionne ledit lot d'instruments et on traite simultanément ledit chariot démonté et le lot d'instruments dans un ensemble de désinfection et de stérilisation.

Ce procédé pour la mise en oeuvre de l'équipement est de plus caractérisée en ce qu'on prévoit un poste d'emmagasinage des éléments de chariots démontés, un poste de montage des chariots, un poste d'emmagasinage de lots d'instruments préconditionnés, en stock stérile, situés dans une seule et même zone stérile, un poste de démontage des chariots et un poste de lavage situés dans une seule et même zone dite zone sale, au voisinage d'un ensemble de désinfection et de stérilisation disposé entre ladite zone stérile et la zone sale et isolé de celles-ci de façon connue.

On comprend que grâce à l'équipement, et au procédé suivant l'invention on peut éliminer de la salle d'opérations tous les appareils ou installations qui peuvent être rendus mobiles, c'est-à-dire d'une part tous les éléments de rangements: tiroirs, placards, vitrines, catgutiers, ainsi que les guichets remplacés par l'équipement suivant l'invention, et d'autre part le système d'aspiration chirurgien (niche à flacons), le bistouri électrique, et les paillasses éventuelles, seuls restant fixes: le négatoscope, l'éclairage chirurgical et les prises électriques et de gaz médicaux.

Le négatoscope et les prises pouvant être facilement réalisés étanches, la salle d'opérations est ainsi pratiquement dépourvue de toutes les installations murales utilisées jusqu'à présent, ce qui permet de réaliser la salle avec des murs parfaitement lisses et de ce fait pouvant être très facilement nettoyés et désinfectés.

En outre la construction du bloc opératoire et de la salle d'opérations en sont considérablement simplifiés ce qui permet d'abaisser de façon importante le prix de revient global de l'installation.

La suppression du stockage au niveau de la salle d'opérations entraîne bien entendu la nécessité de préparer le matériel nécessaire à chaque intervention au niveau du stock stérile.

En conséquence, avec l'équipement et le procédé suivant l'invention, on préparera à l'avance dans le local d'emmagasinage en stock stérile des paniers adaptés pour être utilisés avec les chariots et contenant des lots de matériel, d'instrumentations et d'accessoires nécessaires pour des interventions déterminées, constituant ainsi des ensembles répertoriés préparés à l'avance et emmagasinés à l'état stérile, prêts à être immédiatement utilisés.

L'installation suivant l'invention prévoit dans ce but un local situé en zone dite "propre" comportant des éléments spéciaux de support et d'emmagasinage pourvus de moyens identiques à ceux des chariots pour soutenir les paniers garnis et permettant ainsi un emmagasinage en stock stérile des ensembles de paniers répertoriés.

D'autres caractéristiques et avantages d'un mode de réalisation de l'invention apparaîtront au cours de la description qui va suivre faite en se référant aux dessins annexés donnés uniquement à titre d'exemple et dans lesquels:

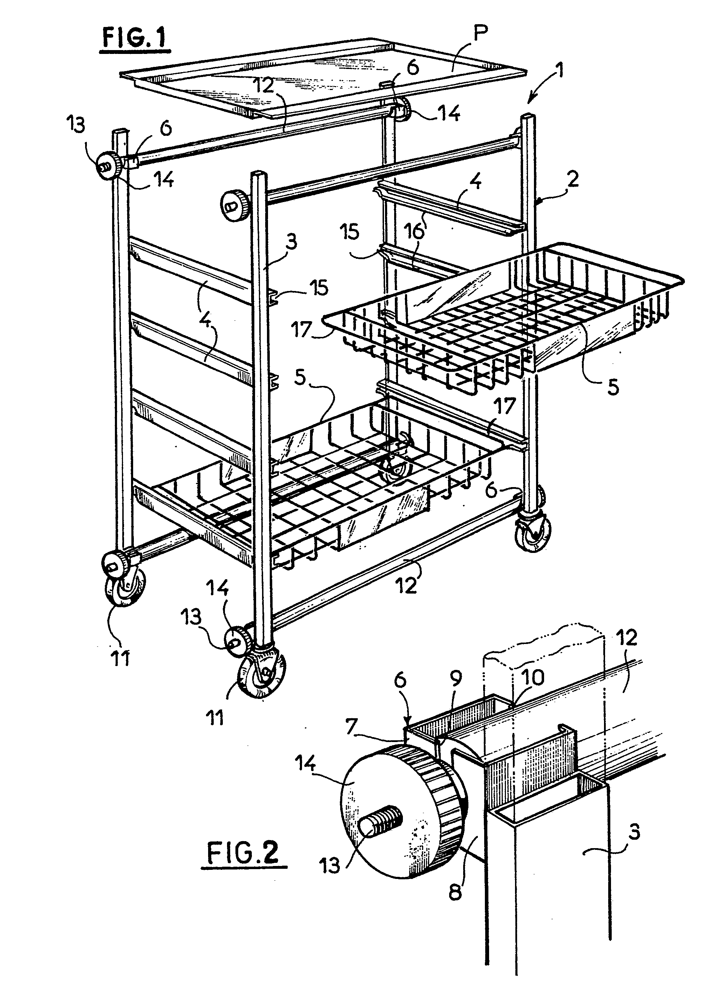

— les Fig. 1 à 5 sont des vues de différents éléments composant l'équipement suivant l'invention pour la mise en oeuvre du procédé, les différentes vues montrant: un chariot démontable suivant l'invention en perspective, un détail du dispositif de fixation rapide des entretoises, pour le montage des chariots, une vue en perspective montrant un chariot en cours de montage sur un dispositif de montage et de démontage, un porte-chariots mobile pour les éléments des chariots démontés, adapté pour être facilement désinfecté et une vue en perspective d'un support fixe pour des paniers contenant des lots répertoriés d'instruments pour différentes opérations, respectivement;

— la Fig. 6 est un schéma synoptique illustrant la séquence des différentes phases du procédé suivant l'invention et montrant la délimitation des trois zones de l'installation;

— la Fig. 7 est un schéma d'ensemble montrant l'implantation de l'installation pour un mode particulier de réalisation du procédé suivant l'invention.

En se référant aux Fig. 1 à 5 on a représenté les différents éléments de l'équipement suivant l'invention. Cet équipement comprend un chariot démontable 1, un dispositif 20 de montage et de démontage des chariots, un porte chariots démontés 33 et un support fixe 41.

La Fig. 1 montre un chariot 1 conçu et adapté spécialement pour être utilisé dans un bloc opératoire chirurgical, comprenant une salle d'opération dont les murs sont dépourvus de volumes intérieurs de rangement, ce chariot étant constitué par deux éléments d'extrémité identiques 2 comprenant chacun deux montants parallèles 3 reliés entre eux par des traverses espacées 4 parallèles constituant des supports pour des paniers 5 comme on le décrira dans la suite.

Les montants 3 des éléments d'extrémité, qui sont de façon commode constitués par des tubes creux en acier inoxydable de section rectangulaire et fermés à leurs extrémités, comportent sur leurs faces en regard, au voisinage de leurs extrémités supérieures et inférieures, des moyens de fixation 6 dont l'un est représenté à plus grande échelle à la Fig. 2.

Ces moyens de fixation comprennent chacun une ferrure 7 repliée de façon à délimiter un parallélépipède à peu près cubique ouvert à sa partie supérieure et fixé, par exemple soudé par une face latérale sur la face du montant 3 dirigée vers la face correspondante du montant opposé symétrique. Le cube délimité par la ferrure 7 dont la face supérieure est ouverte comporte dans l'une 8 de ses faces latérales adjacentes à la face supérieure une fente 9 s'étendant verticalement à partir de l'arête supérieure du cube 7 et se terminant à peu près au centre de la face 8.

Dans sa face opposée à la face 8, le cube 7 comporte une ouverture 10 de forme semi-circulaire dont l'extrémité arrondie est tangente à la face inférieure du cube 7 et s'ouvrant dans l'arête supérieure de ce cube.

Les moyens de fixation 6 sont disposés sur chaque montant 3 en étant orientés dans le même sens.

Les éléments d'extrémité 2 comportent enfin une roulette orientable 11 fixée à une extrémité de chaque montant 3 au-dessous d'un dispositif de fixation 6, à l'opposé de sa face ouverte. En d'autres termes, les faces supérieures ouvertes des moyens de fixation sont orientées vers le haut.

Deux éléments d'extrémité 2 identiques sont disposés face à face et reliés ensemble au moyen de quatre entretoises identiques 12 reliant chacune les moyens de fixation 6 correspondant de deux éléments d'extrémité opposée.

Chaque entretoise 12 est constituée par une barre qui est par exemple en acier inoxydable, de section avantageusement cylindrique dont le diamètre est égal à la largeur de l'ouverture 10 de chaque moyen de fixation 6. Chaque entretoise 12 est fermée à son extrémité et comporte une tige axiale centrale 13 filetée en saillie vers l'extérieur sur laquelle est vissé un disque 14 ayant de préférence un diamètre supérieur à celui de la barre 12 et également de préférence en acier inoxydable.

Comme on l'a représenté à la Fig. 2, la barre constituant chaque entretoise 12 est disposée avec ses extrémités introduites respectivement dans une ouverture 10 de chaque ferrure cubique 7 avec sa tige filetée 13 disposée dans la fente 9 et faisant saillie à l'extérieur de la paroi 8, le disque 14 formant poignée étant disposé à l'extérieur de la paroi 8. On comprend que l'extrémité de chaque entretoise se trouve alors disposée contre la face interne de la paroi 8. Les entretoises 12 étant maintenues sans jeu dans les ferrures 7 il suffit de serrer chaque poignée 14 pour obtenir un assemblage parfaitement rigide des deux éléments d'extrémité 2 du chariot 1 de façon extrêmement simple et rapide, sans aucun outil et par des moyens simples, mettant ainsi le montage des chariots 1 à la portée d'une infirmière ou d'une assistante, même sans aucune connaissance ni force physique particulière, un serrage suffisant étant assuré sans effort par un filetage des tiges 13 ayant un pas approprié.

Chacune des traverses 4 reliant ensemble deux montants 3 est constituée avantageusement par une glissière fixée sur les montants 3 de façon à présenter deux ailes horizontales s'étendant par rapport aux ferrures 7 du même côté que les ouvertures 10 de celles-ci. Les extrémités 15 de chaque glissière 4 sont légèrement relevées et repliées vers l'extérieur comme on le voit à la Fig. 1 de façon à délimiter entre elles une partie horizontale rectiligne 16 parallèle au plan contenant les extrémités 15 et légèrement espacées de ce plan.

Les parties 16 horizontales des traverses 4 qui se font face vers l'intérieur constituent des supports adaptés pour recevoir les paniers 5.

Chaque panier 5 est constitué par une grille en fil d'acier inoxydable ayant deux parties horizontales d'extrémité 17 formant poignée, la largeur de chaque panier, et également de chacune de ses poignées 17, étant égale à la longueur de la partie horizontale 16 de chaque traverse 4, entre les extrémités 15 de celle-ci, la longueur de chaque panier 5 étant à peu près égale à la distance entre les éléments d'extrémité 2, déterminés par la longueur des entretoises 12.

On comprend que grâce à cet agencement, il est facile d'introduire horizontalement un panier 5 dans un chariot 1 monté de façon que ses poignées 17 se trouvent soutenues par les parties 16 des traverses 4, les extrémités des poignées 17 étant arrêtées par les extrémités 15 formant butées, en saillie vers le haut. Le panier se trouvant en place peut être facilement retiré en le soulevant légèrement pour échapper aux butées d'extrémité 15, d'un côté ou de l'autre du chariot tout en étant parfaitement maintenu sans risque de tomber ou de glisser lorsque le chariot est déplacé sur ses roulettes, ou lorsqu'un panier est partiellement tiré, comme un tiroir.

De préférence certaines des roulettes orientables 11 comportent un dispositif de freinage pouvant être actionné au pied (non représenté) d'un type connu classique.

Chaque chariot est enfin complété par un plateau P de forme rectangulaire également en acier inoxydable, présentant une forme légèrement creuse et reposant sur les deux entretoises 12 supérieures, qui le maintiennent entre elles et entre les extrémités supérieures des montants 3.

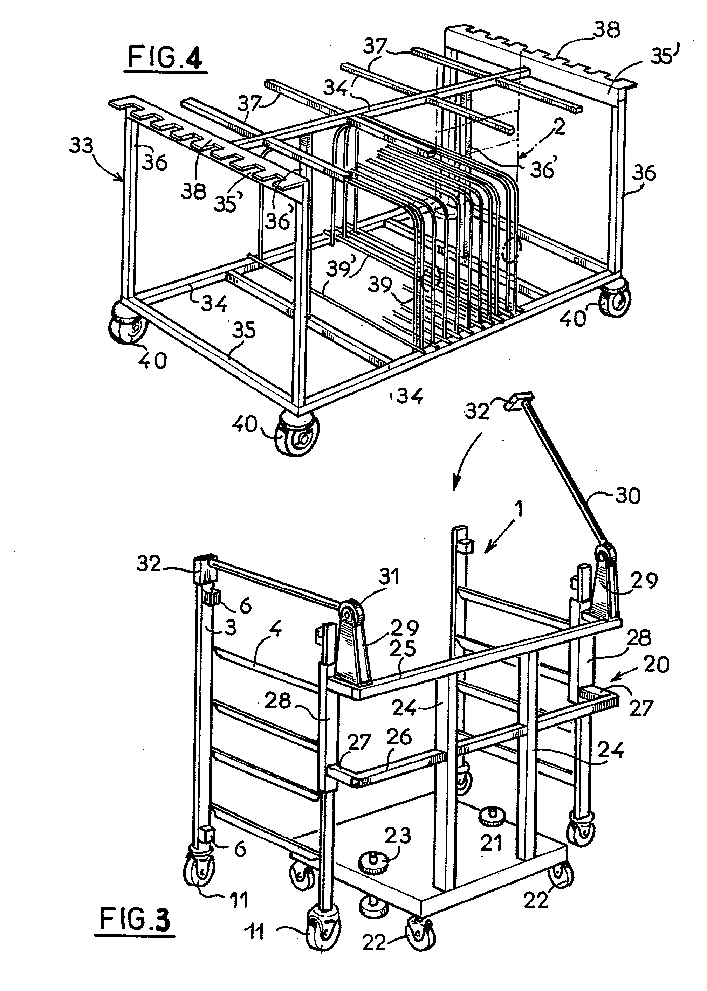

Les moyens de fixation 6 voisins des roulettes 11 sont situés à une distance suffisante au-dessus de celles-ci pour permettre d'amener le chariot au-dessus de la partie inférieure d'un dispositif de montage ou de démontage désigné dans son ensemble par la référence 20 à la Fig. 3.

Le dispositif de montage 20 comprend un plateau 21 de forme à peu près rectangulaire monté sur quatre roulettes orientables 22 et est pourvu latéralement d'au moins deux vérins à vis 23 d'immobilisation.

Sur l'un de ses côtés, le plateau 21 comporte deux montants verticaux parallèles 24 espacés sur les extrémités desquels est fixée une traverse horizontale supérieure 25 dont la longueur est égale à la longueur d'un chariot 1.

Une seconde traverse horizontale 26 d'une longueur égale à la traverse 25 est fixée parallèlement entre la traverse 25 et le plateau 21.

Des bras horizontaux 27 de même longueur sont fixés à angle droit aux extrémités des traverses 25 et 26 de façon à s'étendre parallèlement dans le même sens.

Les extrémités des bras 27 situées d'un même côté par rapport aux montants 24 sont reliées entre elles par des fers verticaux 28 de section en U disposés de façon que leurs ailes s'étendent parallèlement dans le même sens que les bras 27.

Un support 29 est fixé sur chaque extrémité de la traverse horizontale 25 et sur une partie du bras 27 correspondant, et un bras rabattable 30 est articulé par une extrémité au sommet de chaque support 29 au moyen d'un axe 31. A son extrémité opposée au support 29 chaque bras 30 comporte un organe de retenue 32 constitué par un élément creux présentant en section une forme rectangulaire, ouvert vers le bas et dont la section est un peu supérieure à celle d'un montant 3 du chariot 1.

D'une façon avantageuse le dispositif 20 est entièrement réalisé en acier inoxydable et ses roulettes orientables comportent un dispositif de freinage.

Ce dispositif permet de monter facilement un chariot tel que décrit plus haut: on dispose deux éléments d'extrémité 2 face à face verticalement en introduisant un montant 3 dans chacune des ferrures verticales 28 de section en U, et on rabat le bras 30 correspondant de façon que l'organe de retenue 32 vienne coiffer l'extrémité supérieure du montant 3 opposé à celui maintenu dans la ferrure 28. Les deux éléments d'extrémité 2 se trouvent ainsi maintenus face à face verticalement à la distance voulue l'un de l'autre. On dispose alors les entretoises supérieures 12 en place dans les dispositifs de fixation 6 en les faisant passer au-dessous des bras 30 et en serrant simultanément avec chaque main les poignées d'extrémité 14 des entretoises. On dispose ensuite de la même façon les entretoises inférieures 12 en les faisant passer entre la traverse inférieure 4 et le plateau 21 du dispositif 20, le chariot 1 se trouvant ainsi complètement monté et on le libère en relevant les bras 30.

On comprend que cette opération extrêmement simple et rapide peut être effectuée facilement par un personnel non qualifié.

Pour démonter un chariot on effectue les mêmes opérations en sens inverse.

Lorsqu'un chariot est démonté les six éléments qui le constituent, savoir les deux éléments d'extrémité 2 et les quatre entretoises 12 peuvent être disposés sur un porte-chariots déplaçable désigné dans son ensemble par la référence 33 à la Fig. 4.

Le porte-chariots 33 comprend des moyens pour suspendre et retenir les éléments de plusieurs chariots démontés.

Ces moyens comprennent un bâti constitué par un cadre inférieur rectangulaire comportant deux longerons 34 reliés par quatre traverses 35. Ce cadre inférieur comporte à ses extrémités deux montants verticaux parallèles 36 réunis à leur extrémité supérieure par une traverse 35'.

Les milieux des traverses 35' sont reliés entre eux par un longeron 34' soutenu par deux montants intermédiaires 36' et portant deux paires espacées de bras horizontaux parallèles transversaux 37.

Les bras 37 de chaque paire sont espacés d'une distance un peu inférieure à la longueur des traverses 4 des éléments d'extrémité 2 des chariots.

Le porte-chariots, déplaçable 33 comprend en outre à chaque extrémité un râtelier constitué par une bande de métal crénelée 38 fixée horizontalement sur la partie supérieure de chaque traverse 35' de façon que les pattes délimitant les créneaux s'étendent horizontalement vers l'extérieur.

Le porte-chariots comprend enfin des moyens de retenue verticaux 39 constitués par des tiges de métal recourbées en U fixées verticalement et parallèles par les extrémités de leurs branches dans une partie intermédiaire de la longueur des longerons 34, des tiges transversales 39' étant disposées entre les extrémités de chaque tige 39.

Le porte-chariots 33 comme le chariot 1 est entièrement réalisé en éléments d'acier inoxydable et comporte aux angles de son cadre inférieur des roulettes orientables 40 qui sont également de façon avantageuse du type muni d'un frein.

Le porte-chariots déplaçable 33 est destiné à ranger et à porter avec le plus faible encombrement possible des éléments d'un certain nombre de chariots.

Les éléments d'extrémité 2 des chariots sont suspendus sur les paires de bras 37 de part et d'autre du longeron 34' sur lesquels ils sont soutenus par la traverse supérieure 4 de chaque élément, les entretoises 12 sont suspendues verticalement aux râteliers 38 dans les créneaux desquels sont introduites les tiges filetées 13, les poignées 14 étant serrées pour les maintenir en place.

Les plateaux supérieurs amovibles P des chariots sont disposés verticalement entre les organes de retenue 39.

On comprend que grâce à cet agencement un grand nombre de chariots démontés peut être disposé sur un seul porte-chariots 33.

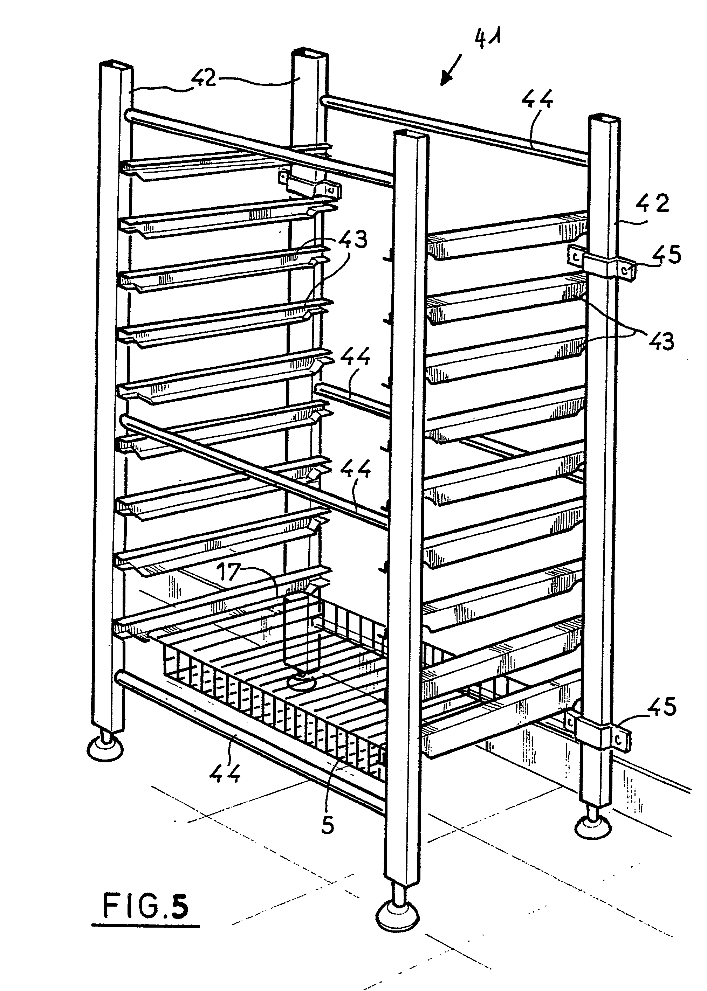

Le dernier élément de l'équipement suivant l'invention est constitué par un support fixe désigné dans son ensemble par la référence 41 et représenté à la Fig. 5, destiné à être installé dans le stock stérile de l'installation qui sera décrite dans la suite.

Le support fixe 41 est simplement constitué de deux éléments d'extrémité verticaux parallèles 42 comportant des traverses horizontales parallèles 43 qui sont identiques aux montants 3 et aux traverses 4 des éléments d'extrémité 2 des chariots, et pouvant ainsi être fabriqués simultanément avec ceux-ci, deux montants 42 correspondants pouvant être fixés le long d'un mur par des moyens quelconques appropriés tels que des ferrures 45.

Les traverses 43 sont ainsi adaptées pour recevoir des paniers tels que les paniers 5 décrits plus haut disposés les uns au-dessus des autres avec un espacement 44 juste suffisant pour permettre l'introduction et le retrait des paniers individuellement.

Les supports fixes 41 sont destinés à être montés fixes dans le stock stérile dans lequel sont emmagasinés les paniers garnis de lots d'instruments comme indiqué plus haut en référence au procédé suivant l'invention.

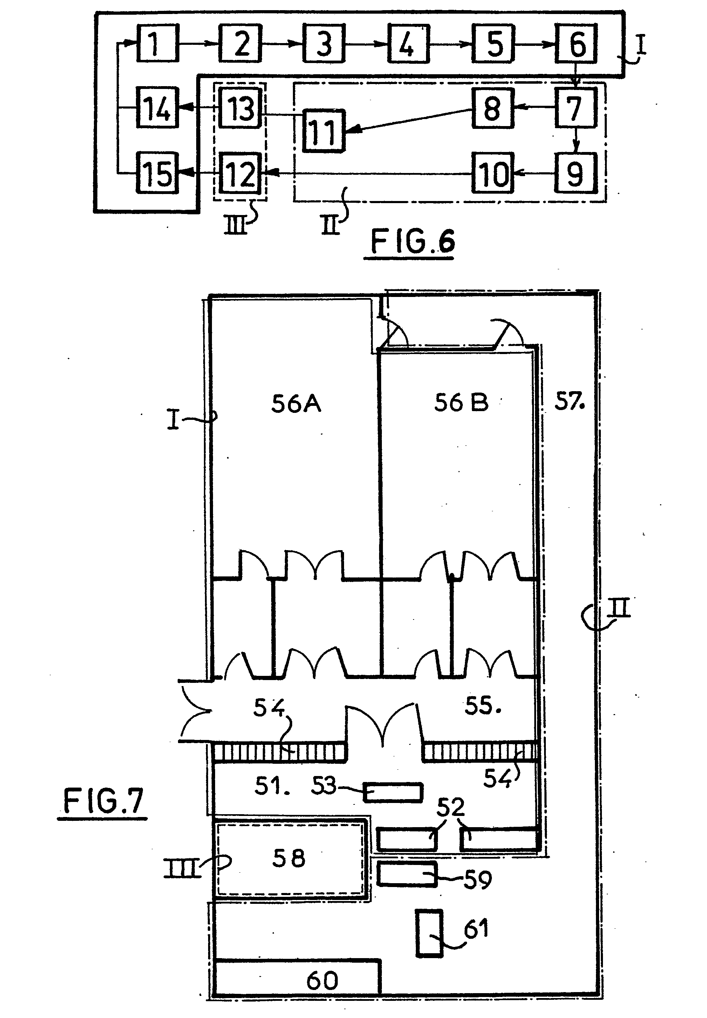

En se référant maintenant à la Fig. 6 qui montre la séquence des différentes phases dans la mise en oeuvre du procédé suivant l'invention.

La zone I délimitée en traits pleins représente la zone dite "propre", c'est-à-dire stérile. Dans la phase I du procédé, on prélève dans une salle d'emmagasinage en stock stérile des éléments de chariots démontés disposés sur un porte-chariots déplaçable, on dispose dans la phase 2, les éléments de chariots sur un dispositif de montage (Fig. 4) et on assemble les différents éléments de chariots.

Dans la phase 3 on prélève dans des supports fixes (Fig. 6) montés dans la salle d'emmagasinage en stock stérile un certain nombre de paniers contenant chacun une partie des instruments, accessoires et autres, préalablement répertoriés, nécessaires pour un chirurgien pour effectuer une intervention donnée et, dans la phase 4, on charge ces paniers sur les chariots montés.

Dans la phase 5 du procédé on transporte ensuite ces chariots chargés en salles d'opérations. Le chirurgien effectue alors son intervention en se servant des instruments et des accessoires contenus dans les paniers en utilisant les chariots comme guéridons de service, dans la phase 6.

Dans la phase 7 le personnel de salle sort les chariots de la salle d'opérations et les amène dans la stérilisation côté "sale" délimitée en traits mixtes et désignée à la Fig. 1 par la référence II dans laquelle, dans la phase 8, on décharge les paniers des chariots, on dispose ces derniers dans un dispositif de démontage dans la phase 9 et on les démonte, en phase 10 on dispose ensuite les différents éléments des chariots sur un porte-chariots déplaçable adapté pour porter les éléments de plusieurs chariots démontés, et dans la phase 11 on procède au lavage des paniers ainsi que du matériel qu'ils contiennent et on reconditionne les lots d'instruments dans les paniers.

On effectue la phase 12 dans une zone délimitée en traits interrompus à la Fig. 1 et désignée par la référence III qui représente un ensemble de désinfection et de stérilisation, phase dans laquelle on désinfecte les porte-chariots démontés et dans la phase 13 on stérilise les paniers et leur contenu.

Dans les deux dernières phases, 14 et 15 du procédé on emmagasine dans la phase 14 les paniers stériles sortant de l'ensemble de stérilisation dans la salle de stockage stérile et on les dispose dans les supports fixes prévus dans cette salle tandis que dans la phase 15 on emmagasine dans ladite salle de stockage les porte-chariots déplaçables portant les éléments de chariots désinfectés, à l sortie de l'ensemble de désinfection, prêts à être réutilisés dans une nouvelle séquence des phases du procédé.

On constate à l'énumération des différentes phases du procédé qu'à aucun moment il n'est nécessaire d'utiliser un matériel, des instruments ou des accessoires d'une provenance extérieure; la totalité du matériel nécessaire aux chirurgiens et à ses aides arrivant en salles d'opérations en provenant du stock stérile et ressortant en totalité de la salle d'opérations pour être reconditionné en vue d'une nouvelle opération. •

En d'autres termes on comprend que grâce au procédé suivant l'invention la totalité du matériel nécessaire à un chirurgien et à ses aides pour effectuer une intervention donnée parcourt un circuit prévu dans une installation en transitant au cours d'une seule des phases (phase 6) du procédé par la salle d'opérations d'où elle ressort après l'opération pour revenir à son point de départ.

On décrira maintenant en référence à la Fig. 7 un schéma d'une installation pour une mode particulier de réalisation du procédé suivant l'invention.

En se référant à la Fig. 7 l'installation comprend une zone dite stérilisation côté "propre" ou stock stérile désignée par la référence I et délimitée en traits pleins, une zone dite stérilisation côté "sale" désignée par la référence II et délimitée en traits mixtes, et une zone intermédiaire III délimitée en traits interrompus et qui est constituée par un ensemble de désinfection et de stérilisation.

La zone propre I comprend un stock stérile 51 dans lequel est installé un poste 52 d'emmagasinage des éléments de chariots démontés et désinfectés, ce poste étant constitué par un porte-chariots déplaçable 33 décrit plus haut qui est disposé à l'emplacement le plus pratique dans le stock stérile 51, emplacement dans lequel il est immobilisé grâce au système de freinage de ses roulettes. Ce poste d'emmagasinage 52 est celui dans lequel sont prélevés des éléments de chariots dans la phase I du procédé décrit plus haut.

Le stock stérile 51 comporte également un poste 53 de montage des chariots, constitué par un dispositif de montage 20 tel que décrit plus haut, immobilisé au moyen des dispositifs de freinage de ses roulettes, ce poste de montage 53 étant celui utilisé dans la phase 2 du procédé précité.

Il est en outre prévu dans le stock 51 au moins un poste 54 de stockage des paniers garnis de leurs lots d'instrumentation répertoriés stérilisés, ce poste étant constitué par au moins un support fixe tel que le support 41 décrit plus haut. Ce poste 54 est celui utilisé dans la phase III du procédé.

La salle 51 peut s'ouvrir sur un couloir 55 desservant par exemple deux salles d'opérations 56a et 56b par l'intermédiaire des locaux habituels utilisés pour la préparation du chirurgien et de ses assistants et d'une chambre d'anesthésie suivant un agencement classique ne faisant point partie de l'invention.

Les salles d'opérations comprennent des portes de sortie qui débouchent sur un couloir 57 qui fait partie de la zone dite sale, ce couloir conduisant à l'entrée 58 de la zone III constituée par l'ensemble de désinfection et de stérilisation.

Il est prévu dans le couloir 57 au voisinage de l'entrée 58 de la zone III un poste 59 de démontage des chariots, constitué par un dispositif tel que le dispositif 20, immobilisé au moyen des systèmes de freinage de ses roulettes au voisinage d'un poste de lavage 60, ce poste 59 de démontage des chariots étant celui qui est utilisé pour la phase 9 du procédé décrit plus haut, et qui est également utilisé avantageusement pour la phase 8 de ce procédé, il est en effet logique d'amener directement le chariot monté au poste de démontage avant de décharger les paniers comme prévu dans la phase 8 du procédé.

Il est également prévu à l'extrémité du couloir un poste 61 de chargement des éléments de chariots démontés, ce poste 61 étant constitué par un porte-chariots déplaçable 33 immobilisé par freinage de ses roulettes au voisinage de l'entrée 58 de l'ensemble de désinfection et de stérilisation. Ce poste 61 est celui utilisé pour la phase 10 du procédé.

Le poste de lavage 60 n'est mentionné qu'à titre indicatif, ce poste existant déjà en un point ou un autre d'un bloc opératoire quelconque au voisinage de l'ensemble de désinfection et de stérilisation, qui est également classique. On notera cependant que ce poste de lavage et cette installation de désinfection bien que ne faisant pas partie de l'invention s'inscrivent cependant tout naturellement dans le procédé de traitement de matériels chirurgicaux suivant l'invention et trouvent bien entendu leur place dans l'installation pour la mise en oeuvre de ce procédé.

On notera toutefois que l'ensemble de désinfection et de stérilisation constituant la zone III doit être prévu de manière à pouvoir recevoir au moins un porte-chariots déplaçable 33 tel que décrit plus haut portant les éléments de plusieurs chariots démontés pour y être désinfectés.

Le poste de lavage 60 est utilisé normalement pour l'exécution de la phase 11 du procédé, tandis que l'ensemble de désinfection et de stérilisation de la zone III est utilisé pour l'exécution des phases 12 de désinfection du porte-chariots déplaçable portant les éléments de chariots démontés et de la phase 13 de stérilisation des paniers reconditionnés.

La phase 14 du procédé, c'est-à-dire l'emmagasinage des paniers stériles avec des lots d'instrumentation et d'accessoires qu'ils contiennent est effectué au sortie de l'ensemble de stérilisation de la zone III, au poste 54 d'emmagasinage déjà utilisé lors de la phase 3, tandis que la phase 15 d'emmagasinage des chariots est effectuée au poste 52 d'emmagasinage utilisé dans la phase 1. On comprend en effet que les porte-chariots déplaçables portant les éléments de chariots démontés doivent être déplacés de temps à autre. En effet lorsqu'un porte-chariots 33 se trouvant au poste 53 de montage a été entièrement dégarni des éléments qu'il portait il convient de le remener dans la zone sale II, au poste 61 pour remplacer celui qui a été complètement chargé d'éléments de chariots et qui est passé dans la salle 51 par l'intermédiaire de la zone III dans laquelle il a été désinfecté. Ce transfert peut être effectué soit entre deux interventions en traversant l'une des salles d'opérations 56A ou 56B pour être ramené par le couloir 57, soit encore en lui faisant traverse l'ensemble de désinfection en sens inverse.

Il ressort de ce qui précède que l'équipement et le procédé pour la mise en oeuvre de cet équipement suivant l'invention permettent un traitement rationnel de l'instrumentation avec un matériel d'une utilisation extrêmement simple et présentant toute facilités en ce qui concerne les possibilités de désinfection et de stérilisation. Grâce à l'invention il est désormais possible de réaliser des salles d'opération à murs parfaitement lisses et dépourvus de toutes solutions de continuité ou de matériels encastrés dont l'installation est coûteuse, les salles d'opérations ainsi réalisées de façon simple et économique pouvant être désinfectées et stérilisées dans leur totalité sans la moindre difficulté tout en abaissant considérablement les frais d'installation des blocs opératoires.

**Revendications**

1. Equipement de stockage de matériel chirurgical spécialement destiné à être utilisé dans un bloc opératoire chirurgical comprenant une salle d'opération dont les murs sont dépourvus de volumes intérieurs de rangement, caractérisé en ce qu'il comprend plusieurs chariots démontables (1) adaptés pour recevoir des paniers (5) et un plateau (P), amovibles, un dispositif (20) de montage desdits chariots (1), un dispositif de démontage (20) desdits chariots et un porte-chariots déplaçable (33) adapté pour recevoir des éléments de plusieurs desdits chariots (1) démontés, dans des positions adjacentes de façon à présenter un encombrement minimal et au moins un support fixe (41) adapté pour recevoir des paniers (5).

2. Equipement suivant la revendication 1, caractérisé en ce que chaque chariot démontable (1) comprend deux éléments d'extrémité identiques (2) réunis par quatre entretoises identiques (12), et des supports pour un plateau supérieur et pour des paniers (5), tous ces éléments étant de préférence en acier inoxydable, et des roulettes orientables (11).

3. Equipement suivant la revendication 2, caractérisé en ce que lesdits éléments d'extrémité (2) comprennent chacun deux montants parallèles verticaux (3) reliés par des traverses (4) constituant des supports pour les paniers (5), et comportant chacun une roulette (11) à une extrémité et des moyens de fixation (6) disposés dans le même sens au voisinage des extrémités de chaque montant 3, et quatre entretoises identiques (12) comportent à leurs extrémités des moyens de fixation rapide adaptés pour coopérer avec les moyens de fixaton (6) des éléments d'extrémité (2) et que les paniers (5) en fil de métal sont adaptés pour être reçu dans les supports constitués par les traverse (4).

4. Equipement suivant la revendication 3, caractérisé en ce que lesdites entretoises (12) sont adaptées pour relier entre elles les extrémités en regard des éléments d'extrémite (2) opposés et sont constituées chacune par une barre ou tige (12) portant à chaque extrémité une tige axiale centrale (13) filetée en saillie vers l'extérieur et un disque (14) formant poignée vissé sur ladite tige 13.

5. Equipement suivant la revendication 3, caractérisé en ce que les traverses (4) comportent chacune un logement délimité par deux saillies (15) formant butées et adapté pour recevoir une extrémité d'un panier (5).

6. Equipement suivant la revendication 3, caractérisé en ce que lesdits moyens (6) de fixation solidaires des montants (3) des éléments d'extrémité (2) sont constitués chacun par une ferrure (7) repliée de façon à délimiter un logement fixé au voisinage d'une extrémité de chaque montant, et comportant une face supérieure ouverte, une face latérale (8) adjacente à la face supérieure ouverte comportant une fente (9) dont la largeur est égale au diamètre de ladite tige filetée (13), et la face latérale opposée à la face (8) comportant une ouverture (10) de forme semi-circulaire dont la largeur est égale à celle de ladite entretoise (12).

7. Equipement suivant la revendication 1, caractérisé en ce que le dispositif de montage (20) comprend une base montée sur des roulettes orientables (22) pouvant être freinées et comportant sur l'un de ses côtés deux montants verticaux parallèles espacés (24) dont les extrémités supérieures sont réunies par une traverse supérieure 25 ayant une longueur égale à celle d'un chariot (1), ladite traverse comportant à ses extrémités deux bras latéraux perpendiculaires parallèles (27) portant chacun une ferrure verticale (28) de section en U, un support (29) fixé à chaque extrémité de la traverse (25) au voisinage du bras (27) et sur lequel, un bras rabattable (30) est articulé au moyen d'un axe (31), l'extrémité libre de chaque bras 30 comportant un organe de retenue (32) adapté pour être engagé sur l'extrémité supérieure d'un montant (3) d'un chariot (1), ce dispositif de montage (20) étant de préférence réalisé en acier inoxydable.

8. Equipement suivant la revendication 1, caractérisé en ce que le porte-chariots (33) comprend un cadre (34, 35) monté sur des roulettes orientables et freinables (40), et un bâti (34', 35', 36, 36') comportant des moyens de support et de retenue adaptés pour recevoir les éléments démontés de plusieurs desdits chariots démontables 1, l'ensemble étant réalisé de préférence en acier inoxydable.

9. Equipement suivant la revendication 8, en combinaison avec la revendication 5, caractérisé en ce que lesdits moyens du porte-chariots adaptés pour soutenir les éléments démontés du chariot comprennent deux paires de bras parallèles horizontaux (37) en porte-à-faux espacés d'une distance un peu inférieure à la largeur d'un élément d'extrémité (2) d'un chariot (1), au moins un râtelier (38) adapté pour coopérer avec la tige (13) et la poignée (14) de plusieurs entretoises (12), et des organes de retenue verticaux (39) espacés adaptés pour recevoir entre eux les plateaux (P) des chariots.

10. Equipement suivant la revendication 1, caractérisé en ce que ledit support fixe (41) est constitué par deux éléments d'extrémité verticaux parallèles (42) espacés et de construction identique à celle des éléments d'extrémite (2) des chariots, lesdits éléments d'extrémité étant reliés entre eux par au moins quatre entretoises (44).

11. Procédé pour la mise en oeuvre de l'équipement tel que défini suivant l'une quelconque des revendications 1 à 10 dans un bloc opératoire chirurgical comportant une salle d'opération dont les murs sont dépourvus de volumes intérieurs de rangement, caractérisé en ce qu'on utilise au moins un chariot démonté et au moins un lot d'instruments et d'accessoires préconditionnés disposés dans un stock stérile situé dans la zone propre du bloc opératoire, et dans lequel, préalablement à l'intervention, on procède au montage d'au moins un chariot sur lequel on charge ledit lot préconditionné, on ramène l'ensemble après l'intervention dans une zone sale, comme connu en soi, on procède au démontage dudit chariot dans cette zone sale, on reconditionne ledit lot d'instruments et on traite simultanément ledit chariot démonté et le lot d'instruments dans un ensemble de désinfection et de stérilisation.

12. Procédé suivant la revendication 11 caractérisée en ce qu'on prévoit un poste (52) d'emmagasinage des éléments de chariots démontés, un poste (53) de montage des chariots, un poste (54) d'emmagasinage de lots d'instruments préconditionnés, en stock stérile, situés dans une seule et même zone stérile, un poste (59) de démontage des chariots et un poste de lavage (60) situés dans une seule et même zone dite zone sale, au voisinage d'un ensemble de désinfection et de stérilisation disposé entre ladite zone stérile et la zone sale et isolé de celles-ci de façon connue.

13. Procédé suivant la revendication 12, caractérisée en ce que le poste 52 d'emmagasinage des éléments de chariots démontés est constitué par un porte-chariots 33 tel que défini suivant l'une quelconque des revendications 8 et 9, le poste 53 de montage des chariots et le poste 59 de démontage des chariots étant tous deux constitués par un dispositif de montage 20 tel que défini suivant la revendication 7, le poste d'emmagasinage des lots d'instruments étant constitué par un support fixe 41 tel que défini suivant la revendication 10.

**Patentansprüche**

1. Einrichtung zum Aufbewahren von chirurgischem Spezialmaterial, das in einen chirurgischem Operationstrakt Verwendung finden soll, zum dem ein Operationssaal gehört, dessen Wände im Inneren keinen Ablageplatz bieten, dadurch gekennzeichnet, daß sie aus mehreren zerlegbaren Wagen (1) die zur Aufnahme von abnehmbaren Körben (5) und einer abnehmbaren Platte (P) ausgebildet sind, ferner aus einer Vorrichtung (20) für den Zusammenbau der Wagen (1), einer Vorrichtung (20) zum Zerlegen der Wagen (1), ferner aus einem beweglichen Wagenträger (33), der so ausgebildet ist, daß er die Bestandteile mehrerer solcher zerlegter Wagen (1) in derartig benachbarter Lage aufnehmen kann, daß ein minimaler Raumbedarf entsteht, sowie aus wenigstens einem feststehenden Gestell (41) besteht, das zur Aufnahme der Körbe (5) ausgebildet ist.

2. Einrichtung nach Anspruch 1, dadurch gekennzeichnet, daß jeder zerlegbare Wagen (1) zwei gleiche Endteile (2), die durch vier gleiche Querstreben (12) und durch Träger für eine Deckplatte und für die Körbe (5) miteinander verbunden sind, wobei alle diese Elemente vorzugsweise aus rostfreiem Stahl bestehen, sowie schwenkbare Rollen (11) aufweist.

3. Einrichtung nach Anspruch 2, dadurch gekennzeichnet, daß die genannten Endteile (2) jeweils zwei parallel verlaufende senkrechte Pfosten (3) aufweisen, die durch die Träger für die Körbe (5) bildende Taversen (4) miteinander verbunden sind und jeweils an einem Ende mit einer Rolle und mit gleichartig angeordneten Befestigungselementen (6) in der Nachbarschaft ihrer Enden versehen sind, daß vier gleiche Querstreben (12) an ihren Enden mit Elementen zur Schnellmontage versehen sind, die mit den Befestigungselementen (6) der Endteile (2) zusammenwirken können, und daß die aus Draht bestehenden Körbe (5) so ausgebildet sind, daß sie von den durch die Traversen (4) gebildeten Trägern aufgenommen werden können.

4. Einrichtung nach Anspruch 3, dadurch gekennzeichnet, daß die Querstreben (12) so ausgebildet sind, daß durch sie die oberen und unteren Enden gegenüberliegender Endteile verbunden werden können, und daß sie jeweils aus einem Stab bzw. einer Stange (12) die an jedem Ende einen achsmittigen, nach außen stehenden Gewindebolzen (13) tragen und Scheiben (14) gebildet sind, die jeweils als Griff auf die Gewindebolzen (13) aufgeschraubt sind.

5. Einrichtung nach Anspruch 3, dadurch gekennzeichnet, daß die Traversen (4) jeweils eine durch zwei Ansätze (15) begrenzte Auflagefläche zur Aufnahme des Endes eines Korbes (5) aufweisen.

6. Einrichtung nach Anspruch 3, dadurch gekennzeichnet, daß die mit den Pfosten (3) fest verbundenen Befestigungselemente (6) jeweils aus einem Beschlag (7) bestehen, der derartig geformt ist, daß er in der Nachbarschaft des Endes eines Pfostens eine fest Auflagefläche bildet, wobei er oben offen ist, eine an die Oberseite angrenzende mit einem Schlitz (9), dessen Breite dem Durchmesser des genannten Schraubbolzens (13) entspricht, versehene Seitenfläche (8) und eine dieser gegenüberliegende Seitenfläche aufweist, die mit einem halbkreisförmigen Ausschnitt (10) versehen ist, dessen Breite derjenigen einer Querstrebe (12) entspricht.

7. Einrichtung nach Anspruch 1, dadurch gekennzeichnet, daß die Vorrichtung (20) für den Zusammenbau eine auf schwenk- und feststellbaren Rollen (22) befestigte Grundplatte aufweist, die auf ihrer einen Seite zwei voneinander beabstandete senkrechte Pfosten (24) trägt, deren obere Enden durch eine obere Traverse (25) verbunden sind, deren Länge der Länge eines Wagens (1) entspricht, und die an ihren Enden mit zwei parallelen, senkrecht zu ihr verlaufenden Seitenarmen (27) versehen ist, die jeweils senkrecht verlaufende U-Eisen (28) tragen, daß an jedem Ende der Traverse (25) in der Nachbarschaft eines der Arme (27) eine Stütze (29) befestigt ist, an der ein umklappbarer Arm (30) mittels einer Achse (31) angelenkt ist, dessen Ende ein Halteelement (32) trägt, das mit dem oberen Ende des Pfostens (3) eines Wagens (1) verriegelt werden kann, und daß die Bestandteile dieser Zusammenbauvorrichtung aus rostfreiem Stahl bestehen.

8. Einrichtung nach Anspruch 1, dadurch gekennzeichnet, daß der Wagenträger (33) einen auf schwenk- und feststellbaren Rollen (40) befestigten unteren Rahmen (34, 35) sowie einen oberen Rahmen (34, 35, 36, 36) aufweist, der mit Mitteln zum Abstützen oder Halten der Bestandteile mehrerer der zerlegten Wagen (1) versehen ist, wobei die Bestandteile des Wagenträgers vorzugsweise aus rostfreiem Stahl bestehen.

9. Einrichtung nach Anspruch 5 und 8, dadurch gekennzeichnet, daß die genannten Elemente zum Abstützen und Halten die Form zweier Paare paralleler, horizontaler, freitragender Arme (37), die weniger voneinander beabstandet sind, als um die Breite eines Endteils (2) eines Wagens (1), ferner die Form wenigstens eines Gestells (38), das dazu ausgebildet ist, mit den Bolzen (13) und den Scheiben (14) mehrerer Querstreben zusammenzuwirken, und die Form von vertikalen voneinander beabstandeten Halteelementen (39) haben, die dazu ausgebildet sind, zwischen sich die Platten (P) der Wagen aufzunehmen.

10. Einrichtung nach Anspruch 1, dadurch gekennzeichnet, daß das feststehende Gestell (41) zwei parallele, vertikale und voneinander beabstandete Endteile (42) aufweist, die den gleichen Aufbau wie die Endteile (2) der Wagen haben und die durch wenigstens vier Querstreben (44) miteinander verbunden sind.

11. Verfahren zum Einsatz einer Einrichtung gemäß einem der Ansprüche 1 bis 10 in einem chirurgischen Operationstrakt, zu dem ein Operationssaal gehört, dessen Wände im Inneren keinen Ablageplatz bilden, dadurch gekennzeichnet, daß wenigstens ein zerlegbarer Wagen und wenigstens ein Satz Instrumente und vorgesehener Hilfsmittel Verwendung finden, die in einem im Sterilbereich des Operationstrakts befindlichen sterilen Lager aufbewahrt sind und in dem man vor dem Eingriff wenigstens einen der Wagen zusammenbaut auf den dann der vorgesehene Satz abgelegt wird, daß nach dem Eingriff das ganze in einen Nichtsteril-Bereich verbracht wird, wo das Zerlegen des Wagens vorgenommen wird, daß dann der Satz Instrumente und Hilfsmittel wieder zusammengepackt und gleichzeitig zusammen mit dem zerlegten Wagen in einer Desinfektions- und Sterilisationseinrichtung behandelt wird.

12. Verfahren nach Anspruch 1, dadurch gekennzeichnet, daß ein Platz (52) zum Lagern der Elemente der zerlegten Wagen, ein Platz (53) für den Zusammenbau der Wagen, ein Platz (54) zum Lagern des vorgesehenen Instrumentensatzes und ein steriler Vorratsplatz vorgesehen werden, die sich alle in ein und demselben Sterilbereich befinden, ferner ein Platz (59) zum Zerlegen der Wagen und ein Platz (60) zum Reinigen vorgesehen werden, die sich in ein und demselben Nichtsterilbereich in der Nachbarschaft einer Desinfektions- und Sterilisationseinrichtung befinden, die zwischen dem Sterilbereich und dem Nichtsterilbereich angeordnet und in bekannter Weise diesen gegenüber isoliert ist.

13. Verfahren nach Anspruch 12, dadurch gekennzeichnet, daß der Platz (52) zum Lagern der Elemente der zerlegten Wagen ein Wagenträger (33) nach einem der Ansprüche 8 und 9, die Plätze für den Zusammenbau (53) und das Zerlegen (59) der Wagen jeweils eine Zusammenbauvorrichtung (20) nach Anspruch 7 und der Platz (54) zum Lagern des Instrumentensatzes ein feststehendes Gestell (41) nach Anspruch 10 ist.

**Claims**

1. Equipment for storing surgical appliances especially for use in a surgical operating unit comprising an operating room whose walls have no interior storage volumes, characterised in that it comprises a plurality of trolleys (1) which are capable of being disassembled and adapted to receive detachable baskets (5) and a detachable tray (P), a device (20) for assembling said trolleys (1), a device (20) for disassembling said trolleys and a movable trolley carrier (33) adapted to receive elements of a plurality of said disassembled trolleys (1) in adjacent positions so as to have a minimum overall size and at least a fixed support (41) adapted to receive the baskets (5).

2. Equipment according to claim 1, characterised in that each trolley (1) capable of being disassembled comprises two identical end elements (2) interconnected by four identical struts (12), and supports for an upper tray and for baskets (5), all these elements being preferably of stainless steel, and castors (11).

3. Equipment according to claim 2, characterised in that said end elements (2) each comprise two vertical parallel uprights (3) interconnected by cross-members (4) constituting supports for the baskets (5) and each including a castor (11) at one end and fixing means (6) disposed in the same direction in the vicinity of the ends of each upright (3), and four identical struts (12) comprising at their ends rapid fixing means adapted to cooperate with the fixing means (6) of the end elements (2) and the baskets (5) of metal wire are adapted to be received in the supports constituted by the cross-members (4).

4. Equipment according to claim 3, characterised in that said struts (12) are adapted to interconnect the confronting ends of the opposed end elements (2) and are each constituted by a bar or rod (12) carrying at each end a screwthreaded central axial rod (13) which projects outwardly and a disc (14) constituting a handle screwed onto said rod (13).

5. Equipment according to claim 3, characterised in that the cross-members (4) each comprise a cavity defined by two projections (15) constituting abutments and adapted to receive one end of a basket (5).

6. Equipment according to claim 3, characterised in that said fixing means (6) mounted on the uprights (3) of the end elements (2) are each constituted by a member (7) which is folded in such manner as to define a cavity fixed in the vicinity of an end of each upright and comprising an open upper side, a lateral side (8) adjacent to the open upper side and having a slot (9) whose width is equal to the diameter of said screwthreaded rod (13), and the lateral side opposed to the side (8) having an opening (10) of semi-circular shape whose width is equal to that of said strut (12).

7. Equipment according to claim 1, characterised in that the assembling device (20) comprises a base mounted on castors (22) capable of being braked and including on one of its sides two spaced-apart parallel vertical uprights (24) whose upper ends are interconnected by an upper cross-member (25) which has a length equal to that of a trolley (1), said cross-member having at its ends two parallel perpendicular lateral arms (27) each carrying a U-section vertical member (28), a support (29) fixed to each end of the cross-member (25) in the vicinity of the arm (27) and on which an arm (30) which is capable of being swung over is pivotally mounted by means of a pin (31), the free end of each arm (30) having a retaining element (32) adapted to be engaged on the upper end of an upright (3) of a trolley (1), this assembling device (20) being preferably made of stainless steel.

8. Equipment according to claim 1, characterised in that the trolley carrier 33 comprises a frame (34, 35) mounted on castors capable of being braked (40), and a frame (34', 35', 36, 36') comprising supporting and retaining means adapted to receive the disassembled elements of a plurality of said trolleys (1) capable of being disassembled, the assembly being preferably made of stainless steel.

9. Equipment according to claim 8, in combination with claim 5, characterised in that said means of the trolley carrier adapted to support the disassembled elements of the trolley comprise two pairs of horizontal parallel arms (37) in overhanging relation and spaced apart a distance a little less than the width of an end element (2) of a trolley (1), at least a rack (38) adapted to cooperate with the rod (13) and the handle (14) of a plurality of struts (12), and spaced-apart vertical retaining elements (39) adapted to receive therebetween the trays (P) of the trolley.

10. Equipment according to claim 1, characterised in that said fixed support (41) is constituted by two spaced-apart parallel vertical end elements (42) and of a construction identical to that of the end elements (2) of the trolleys, said end elements being interconnected by at least four struts.

11. A method for using the equipment such as defined according to any one of the claims 1 to 10 in a surgical operating unit including an operating room whose walls have no interior storage volumes, characterised by the use of at least a trolley capable of being disassembled and at least a batch of pre-packed instruments and accessories disposed in a sterile stock located in the clean zone of the operating unit and in which, prior to the surgical intervention, at least a trolley on which said pre-packed batch has been loaded is assembled, the assembly after the intervention is brought to a soiled zone in the known manner and said trolley is disassmbled in this soiled zone, said batch of instruments is put back into condition and said disassembled trolley and the batch of instruments are simultaneously treated in a disinfecting and sterilizing unit.

12. A method according to claim 11, characterised in that there are provided a station (52) for storing elements of disassembled trolleys, a station (53) for assembling the trolleys, a station (54) for storing batches of pre-packed instruments in a sterile stock located in one and the same sterile zone, a station (59) for disassembling trolleys and a washing station (60) located in one and the same zone as said soiled zone, in the vicinity of a disinfecting and sterilizing unit disposed between said sterile zone and the soiled zone and isolated from the last mentioned zones in the known manner.

13. A method according to claim 12, characterised in that the station (52) for storing elements of disassembled trolleys is constituted by a trolley carrier (33) as defined according to any one of the claims 8 and 9, the station (53) for assembling the trolleys and the station (59) for disassembling the trolleys being both constituted by an assembling device (20) as defined according to claim 7, the station for storing batches of instruments being constituted by a fixed support (41) as defined according to claim 10.

FIG.1

FIG.2

FIG.4

FIG.3

FIG.5

FIG.6

FIG.7

4